Europäisches Patentamt

European Patent Office

Office européen des brevets

(11) Veröffentlichungsnummer: **0 277 620 B1**

(12) **EUROPÄISCHE PATENTSCHRIFT**

(45) Veröffentlichungstag der Patentschrift: **03.06.92**

(51) Int. Cl.⁵: **C07C  215/40**

(21) Anmeldenummer: **88101408.8**

(22) Anmeldetag: **01.02.88**

(54) **Verfahren zur Herstellung von optisch aktivem beta-Methylcholin.**

(30) Priorität: **04.02.87 CH 397/87**

(43) Veröffentlichungstag der Anmeldung:
**10.08.88 Patentblatt  88/32**

(45) Bekanntmachung des Hinweises auf die
Patenterteilung:
**03.06.92 Patentblatt  92/23**

(84) Benannte Vertragsstaaten:
**CH DE FR GB LI SE**

(56) Entgegenhaltungen:

**HOUBEN-WEYL: "METHODEN DER ORGANI-
SCHEN CHEMIE", Band IV/1c, Teil I, 1980,
Seiten 364-365, G. Thieme Verlag, New York,
US**

(73) Patentinhaber: **LONZA AG**

**Gampel/Wallis(CH)**

(72) Erfinder: **Voeffray, Robert, Dr.**
**Florastrasse 37**
**Basel(CH)**

(74) Vertreter: **Weinhold, Peter, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Siegfriedstrasse 8**
**W-8000 München 40(DE)**

EP 0 277 620 B1

## Beschreibung

Die Erfindung betrifft ein neues, vorteilhaftes Verfahren zur Herstellung von optisch aktivem β-Methylcholin.

Optisch aktives β-Methylcholin findet als Ausgangsprodukt z.b. für das Acetylderivat oder das Carbamat Eingang in verschiedenen pharmazeutischen Präparaten [J. of Medicinal Chemistry, Vol 29, Nr.7 (1986), S.1128, oder J.Am.Chem.Soc., Vol.57, (1935), S.2125].
Bisher hat es aber an vorteilhaften Verfahren gemangelt, optisch aktives β-Methylcholin herzustellen. Es war lediglich bekannt, durch Racemattrennung von Dimethylaminoisopropanol mit der teuren (+)-3-Brom-campher-10-sulfonsäure und weitere Umsetzung des optisch aktiven Dimethylaminoisopropanols mit einem Methylhalogenid zum optisch aktiven ß-Methylcholin zu gelangen [J.Am.Chem.Soc., Vol.57 (1935), S.2125 ff].
Eine weitere, sehr aufwendige Methode bestand darin, ausgehend von L-Rhamnose über 10 Stufen zum optisch aktiven β-Methylcholin zu gelangen [Carbohydrate Research 16 (1971), S.455-458].
Keines dieser Verfahren kann dem Anspruch an ein technisch durchführbares, einfaches Verfahren genügen.

Es bestand daher die Aufgabe, ein verbessertes Verfahren zu finden, das die gesamten Nachteile der bekannten Verfahren nicht aufweist.

Die Aufgabe konnte gelöst werden mit einem Verfahren nach Patentanspruch 1, worin optisch aktives 3-Chlor-2-oxy-propyltrimethyl-ammoniumchlorid, sowohl mit einer Base als auch in Gegenwart eines Hydrierkatalysators, mit Wasserstoff behandelt wird und danach gegebenenfalls einer Anionenaustauschstufe unterworfen wird.

Unter dem erfindungsgemässen optisch aktiven β-Methylcholin wird, wo nicht anderweitig erwähnt, das entsprechende optisch aktive β-Methylcholinchlorid verstanden.
Das Chloridion kann aber problemlos z.B. unter Anwendung der üblichen Ionenaustauscher durch andere gängige Anionen, wie Jodid, Bromid oder Hydroxid, ausgetauscht werden.

Die optisch aktiven 3-Chlor-2-oxy-propyltrimethyl-ammoniumchloride, d.h. sowohl die (+)- als auch die (-)-Antipode, sind einfach, über eine Racematspaltung mit Weinsäure, gemäss EP-Appl. 0 157 315, zugänglich.

Als Basen werden erfindungsgemässe zweckmässig Erdalkali- oder Alkalicarbonate, -bicarbonate, -hydroxide oder -alkoholate eingesetzt. Bevorzugte Vertreter sind Kaliumverbindungen, wie Kaliumcarbonat, Kaliumbicarbonat, Kaliumhydroxid, Kaliummethylat oder Kalium-tert.-butylat, da das in der Reaktion entstehende Kaliumchlorid in den vorteilhaft angewendeten niederen aliphatischen Alkoholen, wie Ethanol oder Methanol, als Lösungsmittel sehr schlecht löslich ist.
Es ist aber auch möglich, in Wasser oder in Mischungen von Wasser mit den genannten Alkoholen als Lösungsmittel zu arbeiten.

Um einen vollständigen Umsatz zu erhalten, werden zweckmässig, bezogen auf 1 Aequivalent optisch aktives 3-Chlor-2-oxy-propyltrimethyl-ammoniumchlorid, 1 bis 1,5 Aequivalente, vorzugsweise 1 Aequivalent Base verwendet.

Als Hydrierkatalysatoren können Katalysatoren wie Palladium-, Platin- oder Nickelkatalysatoren angewendet werden.
Zweckmässig wird der Palladiumkatalysator, aufgebracht auf ein Trägermaterial, vorzugsweise in einer Menge von 1 bis 10%, auf Kohle, der Platinkatalysator als Platinoxid und der Nickelkatalysator in Form des Raney-Nickels eingesetzt.
Besonders bevorzugter Katalysator ist Palladium 5% auf Kohle. Zweckmässig setzt man den Katalysator in Mengen zwischen 0,1 und 1 Mol% ein.

Vorteilhaft wird in einem Temperaturbereich von 20 bis 80°C, insbesondere bei 40 bis 60°C, und in einem Druckbereich von Normaldruck bis 40 bar gearbeitet. Besonders vorteilhaft liegt der Druck zwischen 5 und 10 bar. Mit Raney-Nickel als Katalysator kann ebenso problemlos unter Normaldruck gearbeitet werden.

Gegebenenfalls kann auch so vorgegangen werden, dass das optisch aktive 3-Chlor-2-oxy-trimethyl-ammoniumchlorid zuerst mit einer der vorgängig genannten Basen, vorteilhaft bei Raumtemperatur, in einem der vorgängig erwähnten, geeigneten Alkohole als Lösungsmittel, umgesetzt wird und erst anschliessend in einer zweiten Stufe der Behandlung mit Wasserstoff, in Gegenwart eines Hydrierkatalysators, unterzogen wird.

Nach einer Reaktionszeit von erfindungsgemäss 8 bis 20 Stunden, abhängig von Druck, Katalysator und Temperatur, kann das gewünschte optisch aktive β-Methylcholin als Chloridsalz nach üblicher Aufarbeitung in Ausbeuten über 80% erhalten werden.

Eine allfällige Reinigung des betreffenden optisch aktiven β-Methylcholinchlorids kann über eine Umkristallisation aus niederen aliphatischen Alkoholen erfolgen.

Wird das erfindungsgemässe Verfahren mit R-(+)-3-Chlor-2-oxypropyltrimethyl-ammoniumchlorid durchgeführt, resultiert das S-(+)-β-Methylcholinchlorid; wird mit S-(-)-3-Chlor-2-oxy-propyltrimethyl-ammoniumchlorid gearbeitet, resultiert das R-(-)-β-Methylcholinchlorid.

Beispiel 1

Herstellung von S-(+)-β-Methylcholinchlorid aus R-(+)-3-Chlor-2-oxy- trimethyl-ammoniumchlorid

In einem Autoklav wurden 7 g (0,05 Mol Kaliumcarbonat und 0,2 g Pd (10% auf Kohle) zu einer Lösung von 18,9 g (0,1 Mol) R-(+)-3-Chlor-2-oxy-trimethyl-ammoniumchlorid ($[\alpha]_D^{24}$ +28,8 (c = 1,$H_2O$), Smp. 208°C) in 75 ml Methanol zugeben. Der Autoklav wurde geschlossen, 3 mal mit $H_2$ gespült, mit 8 bar $H_2$ aufgepresst und die Mischung während 16 Stunden bei 50°C gerührt. Nach Abkühlen und Oeffnen des Autoklaven wurde das heterogene Gemisch bis auf 53 g eingeengt, das ausgefallene Kaliumchlorid (7,8 g, 104%) und der Katalysator abfiltriert und mit je 2 mal 8 ml Ethanol gewaschen. Das Filtrat und das Wasch-Ethanol wurden eingedampft.

Das Rohprodukt (15,95 G, 103,9%, einheitlich nach NMR) wurde in 65 ml n-Butanol während 1 Std. aufgeschlämmt, die heterogene Mischung bei 5°C abgekühlt (1 Std.), filtriert und mit je 2 mal 5 ml n-Butanol gewaschen.

Die Ausbeute an S-(+)-$\beta$-Methylcholinchlorid betrug 11,8 g = 76,9%.

Smp.: 163-165°C

Nmr: ($d_6$-DMSO, 300 MHz) $\delta$ in ppm

1,11 (d, 3H, $CH_3$;

3,18 (s, 9H, $NMe_3$);

3,15-3,40 (m, 2H);

4,23 (dddd, 1H, H-C(2));

5,82 (s, H, OH).

$[\alpha]_D^{25}$ +38,4 (c = 1, $H_2O$)

## Beispiel 2

### Herstellung von R-(-)-$\beta$-Methylcholinchlorid aus S-(-)-3-Chlor-2-oxy- trimethyl-ammoniumchlorid

In einem Autoklav wurden 10,5 g (0,075 Mol) Kaliumcarbonat und 0,2 g Pd (10% auf Kohle) zu einer Lösung von 18,9 g (0,1 Mol) S-(-)-3-Chlor-2-oxy-trimethyl-ammoniumchlorid ($[\alpha]_D^{24}$ +29,3 (c = 1, $H_2O$), Smp. 212°C) in 75 ml Methanol zugegeben. Der Autoklav wurde geschlossen, 3 mal mit $H_2$ gespült, mit 8 bar $H_2$ aufgepresst und die Mischung während 16 Stunden bei 50°C gerührt. Nach Abkühlen und Oeffnen des Autoklaven wurde das heterogene Gemisch mit 20 ml HCl/MeOH innert 1-5 Min. neutralisiert, bis auf 53 g eingeengt, das ausgefallene Kaliumchlorid (11,5 g, 102,6%) und der Katalysator abfiltriert und mit je 2 mal 8 ml Ethanol gewaschen. Das Filtrat und das Wasch-Ethanol wurden eingedampft.

Das Rohprodukt (15,75 g, 102,6%, einheitlich nach NMR) wurde in 65 ml n-Butanol während 1 Std. aufgeschlämmt, die heterogene Mischung bei 5°C abgekühlt (1 Std.), filtriert und mit je 2 mal 5 ml n-Butanol gewaschen.

Die Ausbeute an R-(-)-$\beta$-Methylcholinchlorid betrug 12,1 g = 78,9%.

Smp.: 163-165°C

$[\alpha]_D^{25}$ +38,4 (c = 1, $H_2O$)

## Beispiel 3

### Herstellung von S-(+)-$\beta$-Methylcholinchlorid aus R-(+)-3-Chlor-2-oxy- trimethyl-ammoniumchlorid

In einem Autoklav wurden 13 g (0,2 Mol) Kaliumhydroxid (86%) und 0,5 g $PtO_2$ zu einer Lösung von 37,8 g (0,2 Mol) R-(+)-3-Chlor-2-oxy-trimethyl-ammoniumchlorid ($[\alpha]_D^{24}$ +28,8 (c = 1, $H_2O$), Smp. 208°C) in 150 ml Methanol zugegeben. Der Autoklav wurde geschlossen, 3 mal mit $H_2$ gespült, mit 10 bar $H_2$ aufgepresst und die Mischung während 16 Stunden bei 55°C gerührt. Nach Abkühlen und Oeffnen des Autoklaven wurde das heterogene Gemisch bis auf 105,6 g eingeengt, das ausgefallene Kaliumchlorid (15,2 g, 102%) und das $PtO_2$ abfiltriert und mit je 2 mal 15 ml Ethanol gewaschen. Das Filtrat und das Wasch-Ethanol wurden eingedampft.

Das Rohprodukt (31,8 g, 103,5%, einheitlich nach NMR) wurde in 120 ml n-Butanol während 1 Std. aufgeschlämmt, die heterogene Mischung bei 5°C abgekühlt (1 Std.), filtriert und mit je 2 mal 12 ml n-Butanol gewaschen.

Die Ausbeute an S-(+)-$\beta$-Methylcholinchlorid betrug 24,9 g = 81,1%.

Smp.: 163-165°C

$[\alpha]_D^{25}$ +38,1 (c = 1, $H_2O$)

## Beispiel 4

### Herstellung von S-(+)-$\beta$-Methylcholinchlorid aus R-(+)-3-Chlor-2-oxy- trimethyl-ammoniumchlorid

In einem Hydrierapparat wurden 7 g (0,05 Mol) Kaliumcarbonat und 3,0 g Raney-Nickel zu einer Lösung von 18,9 g (0,1 Mol) R-(+)-3-Chlor-2-oxy-trimethyl-ammoniumchlorid ($[\alpha]_D^{24}$ +28,8 (C = 1, $H_2O$), Smp. 208°C) in 75 ml Methanol zugegeben. Das Gefäss wurde geschlossen, 3 mal mit $H_2$ gespült und die Mischung während 16 Std. bei 30°C unter 1 bar $H_2$ gerührt.

Nach Abkühlen wurde das ausgefallene Kaliumchlorid (7,4 g, 99,3%) und der Ni-katalysator abfiltriert und mit je 2 mal 8 ml Ethanol gewaschen. Das Filtrat und das Wasch-ethanol wurden eingedampft.

Das Rohprodukt (16,1 g, 104,7%, einheitlich nach NMR) wurde in 65 ml n-Butanol während 1 Std. aufgeschlämmt, die heterogene Mischung bei 5°C abgekühlt (1 Std.), filtriert und mit je 2 mal 5 ml n-Butanol gewaschen.

Die Ausbeute an S-(+)-$\beta$-Methylcholinchlorid betrug 8,7 g = 56,7%.

Smp.: 163-165°C

$[\alpha]_D^{25}$ +38,0 (c = 1, $H_2O$)

**Beispiel 5**

Herstellung von S-(+)-β-Methylcholinchlorid aus R-(+)-3-Chlor-2-oxy- trimethyl-ammoniumchlorid

Zu 9,5 g (50 mMol) (+)-3-Chlor-2-oxy-trimethyl-ammoniumchlorid, gelöst in 27,5 g Methanol, wurde bei Raumtemperatur unter Rühren eine Lösung von 5,8 g (50 mMol) Kalium-tert.-butylat in 16,0 g Methanol zugetropft. Das Gemisch wurde während 3 Std. gerührt, das ausgefallene Kaliumchlorid (3,95 g, 105%) filtriert und 2 mal mit je 5 ml Ethanol gewaschen. Das Filtrat und das Wasch-Ethanol wurden eingedampft.

Das Rohprodukt (9,15 g, 119%) wurden in 50 ml Chloroform aufgenommen, wobei sich nach Schütteln das Produkt allmählich löste, bis auf etwas Kaliumchlorid. Dieses unlösliche Kaliumchlorid (0,05 g, Spuren) wurde filtriert.

Nach Eindampfen des Chloroforms betrug die Ausbeute an R-(+)-Glycidyltrimethyl-ammoniumchlorid 7,5 g = 98%.

Smp.: 119-121°C

NMR: ($d_6$-DMSO, 300 MHz) $\delta$ in ppm

2,69 (dd, 1H, J = 5 und 3 Hz, H-C(3));
2,93 (dd, 1H, J = 5 und 5 Hz, H-C(3));
3,22 (dd, 1H, J = 13 und 8 Hz, H-C(1));
3,23 (s, 9H, -N(CH$_3$)$_3$);
3,57 (dddd, 1H, J = 8/5/3 und 3Hz, H-C(2));
4,04 (dd, 1H, J = 13 und 3 Hz, H-C(1)).

$[\alpha]_D^{24}$ +27,1 (c = 1, H$_2$O)

In einem Autoklav wurden 0,2 g Pd (10% auf Kohle) zu einer Lösung von 6,1 g (40,2 mMol) R-(+)-Glycidyltrimethyl-ammoniumchlorid ($[\alpha]_D^{24}$ +27,1 (c = 1, H$_2$O), Smp. 119-121°C) in 25 ml Methanol zugegeben. Der Autoklav wurde geschlossen, 3 mal mit H$_2$ gespült, mit 6 bar H$_2$ aufgepresst und die Mischung während 16 Stunden bei 55°C gerührt.

Nach Abkühlen und Oeffnen des Autoklaven wurde der Katalysator abfiltriert und mit je 2 mal 4 ml Ethanol gewaschen. Das Filtrat und das Wasch-Ethanol wurden eingedampft.

Das Rohprodukt (6,3 g, 102%, einheitlich nach NMR) wurde in 65 ml n-Butanol während 1 Std. aufgeschlämmt, die heterogene Mischung bei 5°C abgekühlt (1 Std.), filtriert und mit je 2 mal 3 ml n-Butanol gewaschen.

Die Ausbeute an S-(+)-β-Methylcholinchlorid betrug 5,3 g = 86,1%.

Smp.: 163-165°C

$[\alpha]_D^{25}$ +37,2 (c = 1, H$_2$O)

**Patentansprüche**

1. Verfahren zur Herstellung von optisch aktivem β-Methylcholin, dadurch gekennzeichnet, dass optisch aktives 3-Chlor-2-oxy-propyltrimethyl-ammoniumchlorid, sowohl mit einer Base als auch in Gegenwart eines Hydrierkatalysators, mit Wasserstoff behandelt wird und danach gegebenenfalls einer Anionenaustauschstufe unterworfen wird.

2. Verfahren nach Patentanspruch 1, dadurch gekennzeichnet, dass die Zugabe der Base und des Hydrierkatalysators sowie die Behandlung mit Wasserstoff von Beginn der Reaktion an erfolgt.

3. Verfahren nach Patentanspruch 1, dadurch gekennzeichnet, dass stufenweise vorgegangen wird, indem zuerst mit einer Base umgesetzt wird und anschliessend die Behandlung mit Wasserstoff in Gegenwart eines Hydrierkatalysators erfolgt.

4. Verfahren nach mindestens einem der Patentansprüche 1, 2 und 3, dadurch gekennzeichnet, dass als Basen Erdalkali-oder Alkalicarbonate, -bicarbonate, -hydroxide oder -alkoholate angewendet werden.

5. Verfahren nach mindestens einem der Patentansprüche 1, 2, 3 und 4, dadurch gekennzeichnet, dass als Hydrierkatalysatoren Platin-, Palladium- oder Nickelkatalysatoren angewendet werden.

6. Verfahren nach mindestens einem der Patentanspruche 1, 2, 3, 4 und 5, dadurch gekennzeichnet, dass eine Reaktionstemperatur von 20 bis 80°C eingehalten wird.

7. Verfahren nach mindestens einem der Patentansprüche 1, 2, 3, 4, 5 und 6, dadurch gekennzeichnet, dass in einem Druckbereich von 1 bis 40 bar gearbeitet wird.

8. Verfahren nach mindestens einem der Patentanspruche 1, 2, 3, 4, 5, 6 und 7, dadurch gekennzeichnet, dass in Wasser, niederen aliphatischen Alkoholen oder Mischungen von niederen aliphatischen Alkoholen mit Wasser als Lösungsmittel gearbeitet wird.

9. Verfahren nach mindestens einem der Patentanspruche 1, 2, 3, 4, 5, 6, 7 und 8, dadurch gekennzeichnet, dass aus S-(-)-3-Chlor-2-oxy-propyltrimethyl-ammoniumchlorid R-(-)-β-Methylcholin hergestellt wird.

10. Verfahren nach mindestens einem der Patentansprüche 1, 2, 3, 4, 5, 6, 7 und 8, dadurch gekennzeichnet, dass aus R-(+)-3-Chlor-2-oxy-propyltrimethyl-ammoniumchlorid S-(+)-β-Methylcholin hergestellt wird.

**Claims**

1. Process for the preparation of optically active beta-methyl choline, characterized in that optically active 3-chloro-2-oxypropyl trimethyl ammonium chloride is treated with a base as well as with hydrogen in the presence of a hydrogenation catalyst and thereafter, if desired, is subjected to an anion exchange step.

2. Process according to patent claim 1, characterized in that the addition of the base and of the hydrogenation catalyst as well as the treatment with hydrogen are effected from the beginning of the reaction.

3. Process according to patent claims 1, characterized in that the operation is carried out stepwise by effecting firstly the reaction with a base and then the treatment with hydrogen in the presence of a hydrogenation catalyst.

4. Process according to at least one of patent claims 1, 2 and 3, characterized in that as bases alkaline earth or alkali carbonates, bicarbonates, hydroxides or alkoxides are used.

5. Process according to at least one of patent claims 1, 2, 3 and 4, characterized in that as hydrogenation catalysts platinum, palladium or nickel catalysts are used.

6. Process according to at least one of patent claims 1, 2, 3, 4 and 5, characterized in that a reaction temperature of 20 to 80°C is maintained.

7. Process according to at least one of patent claims 1, 2, 3, 4, 5 and 6, characterized in that the operation is carried out in a pressure range of 1 to 40 bar.

8. Process according to at least one of patent claims 1, 2, 3, 4, 5, 6 and 7, characterized in that the operation is carried out using as solvents water, lower aliphatic alcohols or mixtures of lower aliphatic alcohols with water.

9. Process according to at least one of patent claims 1, 2, 3, 4, 5, 6, 7 and 8, characterized in that R-(-)-beta-methyl choline is prepared from S-(-)-3-chloro-2-oxypropyl trimethyl ammonium chloride.

10. Process according to at least one of patent claims 1, 2, 3, 4, 5, 6, 7 and 8, characterized in that S-(+)-beta-methyl choline is prepared from R-(+)-3-chloro-2-oxypropyl trimethyl ammonium chloride.

**Revendications**

1. Procédé de préparation de β-méthylcholine optiquement active, caractérisé en ce que l'on traite avec de l'hydrogène du chlorure de 3-chloro-2-oxypropyltriméthylammonium optiquement actif avec une base et aussi en présence d'un catalyseur d'hydrogénation puis on le soumet éventuellement à une étape d'échange d'anions.

2. Procédé selon la revendication 1, caractérisé en ce que l'addition de la base et du catalyseur d'hydrogénation ainsi que le traitement avec l'hydrogène ont lieu au début de la réaction.

3. Procédé selon la revendication 1, caractérisé en ce que l'on procède par étapes en faisant réagir tout d'abord avec une base puis en effectuant le traitement avec l'hydrogène en présence d'un catalyseur d'hydrogénation.

4. Procédé selon au moins l'une des revendications 1, 2 et 3, caractérisé en ce que l'on utilise comme bases des carbonates, bicarbonates, hydroxydes ou alcoolates alcalinoterreux ou alcalins.

5. Procédé selon au moins l'une des revendications 1, 2, 3 et 4, caractérisé en ce que l'on utilise comme catalyseurs d'hydrogénation des catalyseurs de platine, de palladium ou de nickel.

6. Procédé selon au moins l'une des revendications 1, 2, 3, 4 et 5, caractérisé en ce que l'on maintient une température de réaction de 20 à 80°C.

7. Procédé selon au moins l'une des revendications 1, 2, 3, 4, 5 et 6, caractérisé en ce que l'on opère dans un domaine de pressions de 1 à 40 bars.

8. Procédé selon au moins l'une des revendications 1, 2, 3, 4, 5, 6 et 7, caractérisé en ce que l'on opère dans l'eau, dans des alcools aliphatiques inférieurs ou dans des mélanges d'alcools aliphatiques inférieurs et d'eau en tant que solvant.

9. Procédé selon au moins l'une des revendications 1, 2, 3, 4, 5, 6, 7 et 8, caractérisé en ce que l'on prépare la R-(-)-$\beta$-méthylcholine à partir du chlorure de S-(-)-3-chloro-2-oxy-propyltriméthylammonium.

10. Procédé selon au moins l'une des revendications 1, 2, 3, 4, 5, 6, 7 et 8, caractérisé en ce que l'on prépare la S-(+)-$\beta$-méthylcholine à partir du chlorure de R-(+)-3-chloro-2-oxy-propyltriméthylammonium.